(19) Europäisches Patentamt
European Patent Office
Office européen des brevets

(11) **EP 4 411 602 A1**

(12) **EUROPEAN PATENT APPLICATION**
published in accordance with Art. 153(4) EPC

(43) Date of publication:
**07.08.2024 Bulletin 2024/32**

(21) Application number: **22875790.2**

(22) Date of filing: **12.09.2022**

(51) International Patent Classification (IPC):
**G06N 20/00** (2019.01)     **G16C 20/70** (2019.01)
**G16C 60/00** (2019.01)

(52) Cooperative Patent Classification (CPC):
**G06N 20/00; G16C 20/70; G16C 60/00**

(86) International application number:
**PCT/JP2022/034047**

(87) International publication number:
**WO 2023/053918 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2021 JP 2021159474**

(71) Applicant: **Resonac Corporation
Tokyo 105-7325 (JP)**

(72) Inventors:
• **KAKUDA, Kohsuke**
  **Tokyo 105-8518 (JP)**
• **TAKEMOTO, Shimpei**
  **Tokyo 105-8518 (JP)**
• **TAKASHI, Hiroko**
  **Tokyo 105-8518 (JP)**
• **OKUNO, Yoshishige**
  **Tokyo 105-8518 (JP)**

(74) Representative: **Strehl Schübel-Hopf & Partner
Maximilianstrasse 54
80538 München (DE)**

(54) **PREDICTION MODEL CREATION METHOD, PREDICTION METHOD, PREDICTION MODEL CREATION DEVICE, PREDICTION DEVICE, PREDICTION MODEL CREATION PROGRAM, AND PREDICTION PROGRAM**

(57)     Prediction accuracy of trained prediction models is improved by setting an appropriate weight using a trained clustering model and classified clusters. A method of generating a model for predicting a material characteristic includes a step of acquiring a training dataset, a step of generating a trained clustering model using the training dataset and a clustering model, and classifying the training dataset into N clusters, a step of calculating a distance between centroids of the clusters, a step of calculating a weight between the clusters using the distance between the centroids of the clusters and a parameter representing a feature of the training dataset, and a step of generating, for the N clusters, respective trained prediction models $\{M_i\}_{1 \le i \le N}$ using the clusters and the weight.

FIG.1

**Description**

TECHNICAL FIELD

This application is based upon and claims priority to Japanese Patent Application No. 2021-159474 filed on September 29, 2021, the entire contents of which are incorporated herein by reference.

The present disclosure relates to a prediction model generating method, a prediction method, a prediction model generating device, a prediction device, a prediction model generating program, and a prediction program in consideration of clustering and weighting.

BACKGROUND ART

Conventionally, materials have been designed by repeating trial production based on experiences of material developers. In such a case, extensive experimentation is required to obtain desired characteristics. Therefore, in recent years, attempts have been made to apply machine learning in designing materials. For example, by collecting both design conditions at the time of trial production and evaluation results (characteristic values of materials and the like) of materials manufactured by the trial production, and performing training of a model as a training dataset, characteristic values of materials to be manufactured by the trial production can be predicted under new design conditions with the obtained trained model. This minimizes the number of experiments that need to be performed to obtain the desired characteristics.

RELATED ART DOCUMENT

PATENT DOCUMENT

Patent Document 1: Japanese Laid-Open Patent Publication No. 2020-187417

DISCLOSURE OF THE INVENTION

PROBLEM TO BE SOLVED BY THE INVENTION

For example, Patent Document 1 discloses a method of calculating a physical property prediction value. In the method, training data is classified into clusters, and base models and correction models are obtained, the base model predicting a physical property value using a first predetermined number of pieces of training data close to a representative vector in each cluster, the correction model predicting an inverse of the residual of each base model using a second predetermined number of pieces of training data close to the representative vector. In physical property prediction, for an unknown input vector, the base model and the correction model related to the representative vector close to the unknown input vector are retrieved, a base model prediction value and a correction value prediction value are calculated, and a physical property prediction value is then determined by taking the sum of the base model prediction value and a value obtained by multiplying the correction model prediction value by a predetermined constant value. However, in the case of the physical property prediction method disclosed in Patent Document 1, since the training data included neither in the first predetermined number of pieces of training data nor in the second predetermined number of pieces of training data close to the representative vector is not used for the training of the models, the physical property prediction accuracy may fall, and there is also a problem that over-training is likely to occur.

The present disclosure has been made in view of the concerns set forth above, and an object thereof is to provide a prediction model generating method that improves prediction accuracy using all training data.

MEANS FOR SOLVING THE PROBLEM

The present disclosure includes the following configurations.

[1] A method of generating a model for predicting a material characteristic, the method comprising steps of:

acquiring a training dataset;
generating a trained clustering model, using the training dataset and a clustering model, and classifying the training dataset into N clusters;
calculating a distance between centroids of the clusters;
calculating a weight between the clusters using both the distance between the centroids of the clusters and a parameter representing a feature of the training dataset; and

generating, for the N clusters, respective trained prediction models $\{M_i\}_{1 \leq i \leq N}$ using the clusters and the weight.

[2] A prediction method of a material characteristic to be performed subsequent to the method of generating a model for predicting a material characteristic of [1], the prediction method comprising steps of:

acquiring data used for the predicting;

identifying, using the trained clustering model, that the data used for the predicting belongs to a cluster p among training dataset clusters classified into N pieces; and

determining a prediction value, using a trained prediction model $M_p$ corresponding to the cluster p with the data used for the predicting as an input.

[3] The method of generating a model for predicting a material characteristic as recited in [1], wherein, in the step of generating a clustering model of [1], at least one or a plurality of clustering techniques among a K-means method, a Nearest Neighbor method, a hierarchical clustering method, a Gaussian mixture method, a DBSCAN method, a t-SNE method, and a self-organizing map method is used.

[4] The method of generating a model for predicting a material characteristic as recited in [1], wherein, in the step of calculating a distance between centroids of the clusters of [1], the distance is calculated using at least one or a combination of a plurality of methods among an euclidean distance method, a Manhattan distance method, a Mahalanobis distance method, a Minkowski distance method, a cosine distance method, a shortest distance method, a longest distance method, a centroid method, a group average method, a Ward's method, a Kullback-Leibler divergence, a Jensen-Shannon divergence, a Dynamic time warping, and an Earth mover's distance.

[5] The method of generating a model for predicting a material characteristic as recited in [1], wherein, as the parameter representing the feature of the training dataset, at least one or a plurality of parameters among a systematic error, a standard deviation, a variance, a coefficient of variation, a quantile, kurtosis, and a skewness related to a characteristic value of the training dataset is used.

[6] The method of generating a model for predicting a material characteristic as recited in [1], wherein, in the step of calculating a weight, at least one or a plurality of weighting functions among an exponential function type, a reciprocal function type, and a reciprocal power type is used.

[7] A device of generating a model for predicting a material characteristic, the device comprising:

a clustering model configured to generate a trained clustering model when a training dataset is input, and classify the training dataset into N clusters; and

a weight defining part configured to calculate a distance between centroids of the clusters of the classified clusters, and a weight between the clusters, using both the calculated distance between the centroids of the clusters and a parameter representing a feature of the training dataset; and

a prediction model $\{M_i\}_{i \leq i \leq N}$ configured to generate, for the clusters, respective trained prediction models using the clusters and the weight.

[8] A device of predicting a material characteristic, the device comprising:

the trained clustering model generated by the device of generating a model for predicting a material characteristic of [7], the trained clustering model being configured to identify, when data used for the predicting is input, that the data used for the predicting belongs to a cluster p among clusters classified into N pieces,;

a trained prediction model $M_p$ corresponding to the identified cluster p and generated by the device of generating a model for predicting a material characteristic of [7], the trained prediction model $M_p$ being configured to determine a prediction value with data used for the predicting as an input; and

an output part configured to output the determined prediction value.

[9] A program of generating a model for predicting a material characteristic, the program causing a computer to execute steps of:

acquiring a training dataset;

generating a trained clustering model using the training dataset and a clustering model, and classifying the training dataset into N clusters;

calculating a distance between centroids of the clusters;

calculating a weight between the clusters using both the distance between the centroids of the clusters and a parameter representing a feature of the training dataset; and

generating, for the clusters, respective trained prediction models $\{M_i\}_{i \leq i \leq N}$, using the clusters and the weight.

[10] A program of predicting a material characteristic, the program causing a computer to execute steps of:

acquiring data used for the predicting;
identifying, using the trained clustering model generated by the program of generating a model for predicting a material characteristic of [9], that the data used for the predicting belongs to a cluster p among training dataset clusters classified into N pieces; and
determining, when the data used for the predicting is input, a prediction value, using a prediction model $M_p$ of [9], the prediction model $M_p$ corresponding to the identified cluster p.

EFFECT OF THE INVENTION

The prediction model generated using the prediction model generating method according to the present disclosure can suppress over-training due to shortage of the number of pieces of data by using all training data, and can improve prediction accuracy by introducing a weight reflecting a tendency of data.

BRIEF DESCRIPTION OF THE DRAWINGS

[FIG. 1] FIG. 1 is a diagram illustrating an example of a functional configuration of a prediction model generating device in a training phase and a prediction device in a prediction phase.
[FIG. 2] FIG. 2 is a diagram illustrating a hardware configuration example of the prediction model generating device and the prediction device.
[FIG. 3] FIG. 3 is a flowchart illustrating a flow of a training process.
[FIG. 4] FIG. 4 is a flowchart illustrating a flow of a prediction process.
[FIG. 5] FIG. 5 is an image of an example of a requirement setting screen in a prediction model generating method according to an embodiment.
[FIG. 6] FIG. 6 is a flowchart illustrating a flow of a normal random forest training process in a comparative example.
[FIG. 7] FIG. 7 is a flowchart illustrating a flow of a normal random forest prediction process in the comparative example.
[FIG. 8] FIG. 8 is a diagram illustrating an example of prediction accuracy.

MODE OF CARRYING OUT THE INVENTION

Hereinafter, embodiments will be described with reference to the accompanying drawings. In this specification and the drawings, components having substantially the same functional configuration are denoted by the same reference numerals, and redundant description thereof will be omitted.

<Functional Configurations of Prediction Model Generating Device and Prediction Device>

First, functional configurations of the prediction model generating device and the prediction device will be described. The prediction model generating device will be described using, as an example, a prediction model generating device that generates a prediction model, using a training dataset including design conditions at the time of trial production and characteristic values of a material manufactured by the trial production. The prediction device will be described using, as an example, a prediction device that predicts characteristic values of a material to be manufactured under new design conditions, using a trained prediction model generated by the prediction model generating device.
The prediction model generating device and the prediction device according to the present embodiment are, however, not limited to the above-described applications, and may be used for applications other than material design.

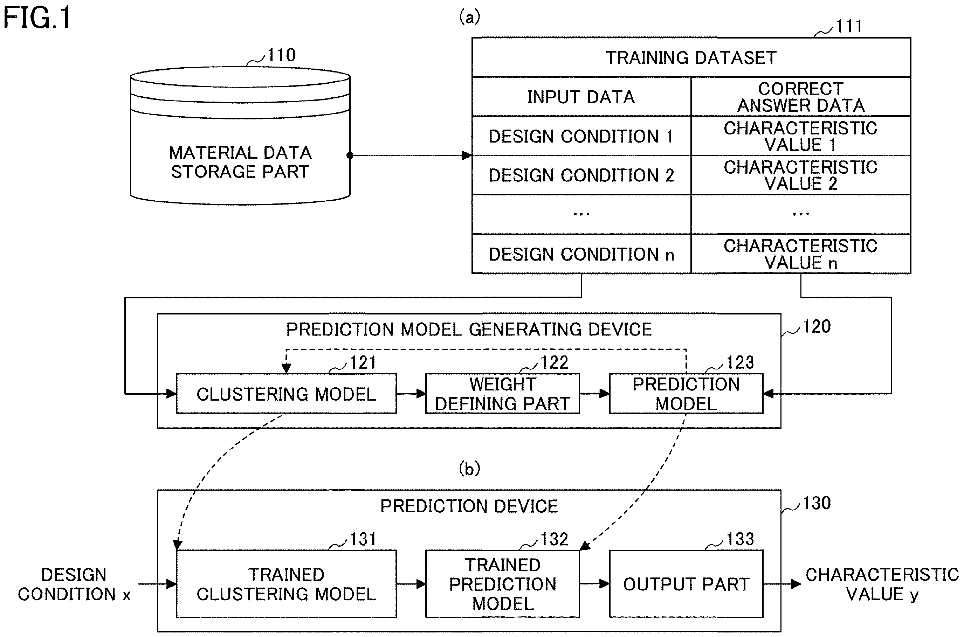

FIG. 1 is a diagram illustrating an example of a functional configuration of the prediction model generating device in a training phase and a prediction device in a prediction phase. A training program is installed in a prediction model generating device 120, and when the program is executed, the prediction model generating device 120 functions as follows (see (a) in FIG. 1):

- a clustering model 121;
- a weight defining part 122; and
- a prediction model 123.

The prediction model generating device 120 trains the clustering model 121 and the prediction model 123 using a training dataset 111 stored in a material data storage part 110, and generates a trained clustering model 131 and a trained prediction model 132.

As illustrated in (a) in FIG. 1, the training dataset 111 includes "input data" and "correct answer data" as information items. The example of (a) in FIG. 1 illustrates a case where "design condition 1" to "design condition n" are stored as "input data" and "characteristic value 1" to "characteristic value n" are stored as "correct answer data".

The clustering model 121 outputs training dataset clusters as output data when "design condition 1" to "design condition n" stored in "input data" of the training dataset 111 are input. That is, the trained clustering model 131 and the training dataset 111 classified into a cluster i are generated when the training dataset 111 is input.

Note that the number of clusters generated by the clustering model 121 is set to N.

Note also that the clustering model 121 to be trained by the prediction model generating device 120 is, for example, a model to be trained based on any one or more of training techniques including the "K-means method, Nearest Neighbor method, hierarchical clustering method, Gaussian mixture method, DBSCAN method, t-SNE method, and self-organizing map method".

More specifically, the clustering model 121 classifies "design condition 1" to "design condition n" stored in "input data" of the training dataset 111 into any cluster i ($1 \leq i \leq N$), and outputs centroid coordinates of the cluster i.

The weight defining part 122 calculates a weight $\{W_{ij}\}_{1 \leq i \leq N, \, 1 \leq j \leq N}$ (where i is greater than or equal to 1 and less than or equal to N and j is greater than or equal to 1 and less than or equal to N") to be used in the prediction model 123, using a distance between the clusters and a parameter representing a feature of the training dataset 111.

A distance $\{l_{ij}\}_{1 \leq i \leq N, \, 1 \leq j \leq N}$ between the classified clusters is represented by the distance between the centroid coordinates of the clusters, and is calculated in N(N-1)/2 ways.

Note that, in the weight defining part 122, the distance between the clusters can be calculated based on any one or a combination of multiple methods including the "euclidean distance method, Manhattan distance method, Mahalanobis distance method, Minkowski distance method, cosine distance method, shortest distance method, longest distance method, centroid method, group average method, Ward's method, Kullback-Leibler divergence, Jensen-Shannon divergence, Dynamic time warping, and Earth mover's distance".

In the weight defining part 122, the parameter representing the feature of the training dataset 111 can be defined using one or more parameters among a "systematic error, standard deviation, variance, coefficient of variation, quantile, kurtosis, and skewness" of "characteristic value 1" to "characteristic value n" stored in "correct answer data".

The weight calculated using the distance between the classified clusters and the parameter representing the feature of the training dataset 111 is represented by a weighting function, and the weighting function is defined using one or multiple types among an "exponential function type, reciprocal function type, and reciprocal power type".

For example, the weighting function $W_{ij}$ can be defined by an exponential function such as a Boltzmann type represented by Equation (1) below.

[Math. 1]

$$w_{ij} = \exp\left(\frac{-l_{ij}}{\tau^{\alpha}}\right) \quad ...(1)$$

where, $l_{ij}$ denotes a distance between clusters, $\tau$. denotes a parameter representing a feature of a training dataset, and $\alpha$ denotes a predetermined constant.

The prediction model 123 is generated by being trained so that when a value determined by multiplying an explanatory variable, included in the training dataset cluster output from the clustering model 121, by the weight, calculated by the weight defining part 122, is input, a response variable (characteristic value) corresponding to the explanatory variable (design condition) used as the input is obtained as output data to output characteristic value.

Note that the prediction model to be trained by the prediction model generating device 120 can use any one or a combination of training techniques including the "random forest, decision tree, gradient boosting, AdaBoost, bagging, linear, partial least squares, Lasso, linear ridge, and elastic net".

Note that in a case where the prediction model generating device 120 trains the prediction model 123, it is assumed that the prediction model 123 $\{M_i\}_{1 \leq i \leq N}$ is trained for N clusters which have been classified by the clustering model 121. That is, training to which the weight $W_{ij}$ is applied is performed with respect to the cluster i, and the trained prediction model 132 $\{M_i\}_{1 \leq i \leq N}$ is generated for each i.

As an example of a training method to which a weight is applied, for example, a method of inputting a weight as a parameter of a fit function of a random forest regression algorithm stored in the scikit-learn can be given.

Accordingly, the prediction model generating device 120 generates the trained clustering model 131 and the trained prediction models 132. The prediction model generating device 120 also applies the generated trained clustering model 131 and trained prediction models 132 to a prediction device 130.

A prediction program is installed in the prediction device 130, and when the program is executed, the prediction device 130 functions as follows (see (b) in FIG. 1) :

- the trained clustering model 131;
- the trained prediction model 132; and
- an output part 133

The trained clustering model 131 is generated by the prediction model generating device 120 performing training of the clustering model 121, using "design condition 1" to "design condition n" stored in "input data" of the training dataset 111.

Furthermore, the trained clustering model 131 identifies that, when data used for performing a prediction (hereinafter, also referred to as predictive data) (design condition x) is input, the predictive data belongs to a cluster p among the N clusters into which the training dataset 111 has been classified.

The trained prediction model 132 is generated for each cluster by the prediction model generating device 120 performing training of the prediction model 123 using the N clusters into which the training dataset 111 has been classified as well as the weight calculated by the weight defining part 122.

The trained prediction model 132 also predicts, when the design condition X and the belonging section p of the cluster output from the trained clustering model, a characteristic value y, using a trained prediction model $132M_p$ corresponding to the belonging section p. The output part 133 outputs the predicted characteristic value as prediction data.

Thus, according to the prediction device 130, sufficient prediction accuracy can be obtained by performing the prediction of the characteristic value using the trained model trained using the cluster to which the design condition x belongs and the weight corresponding to the cluster. That is, according to the present embodiment, the prediction accuracy can be improved in the prediction device using the trained prediction model.

<Hardware Configurations of Prediction Model Generating Device and Prediction Device>

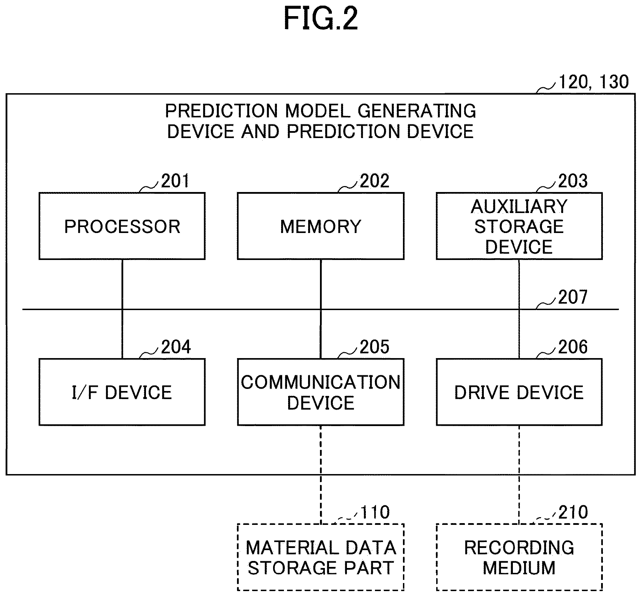

Next, hardware configurations of the prediction model generating device 120 and the prediction device 130 will be described. Since the prediction model generating device 120 and the prediction device 130 have the same hardware configuration, the hardware configurations of the prediction model generating device 120 and the prediction device 130 will be collectively described here with reference to FIG. 2.

FIG. 2 is a diagram illustrating a hardware configuration example of the training device and the prediction device. As illustrated in FIG. 2, each of the training device 120 and the prediction device 130 includes a processor 201, a memory 202, an auxiliary storage device 203, an interface (I/F) device 204, a communication device 205, and a drive device 206. The hardware components of the training device 120 and the prediction device 130 are connected to each other via a bus 207.

The processor 201 includes various computing devices such as a central processing unit (CPU) and a graphics processing unit (GPU). The processor 201 reads various programs (for example, a training program, a prediction program, and the like) on the memory 202 and executes the programs.

The memory 202 includes a main storage device such as a read only memory (ROM) and a random access memory (RAM) . The processor 201 and the memory 202 form what is known as a computer, and the computer implements various functions by the processor 201 executing various programs read out on the memory 202.

The auxiliary storage device 203 stores various programs and various types of data used when the various programs are executed by the processor 201.

The I/F device 204 is a connection device connected to an external device (not illustrated). The communication device 205 is a communication device for communicating with an external device (for example, the material data storage part 110) via a network.

The drive device 206 is a device for setting a recording medium 210. The recording medium 210 includes a medium for optically, electrically, or magnetically recording information, such as a CD-ROM, a flexible disk, or a magneto-optical disk. The recording medium 210 may include a semiconductor memory or the like that electrically records information, such as a ROM or a flash memory.

The various programs installed in the auxiliary storage device 203 are installed by, for example, setting the distributed recording medium 210 in the drive device 206 and reading out the various programs recorded in the recording medium 210 by the drive device 206. Alternatively, the various programs installed in the auxiliary storage device 203 may be installed by being downloaded over a network via the communication device 205.

<Flow of Training Process>

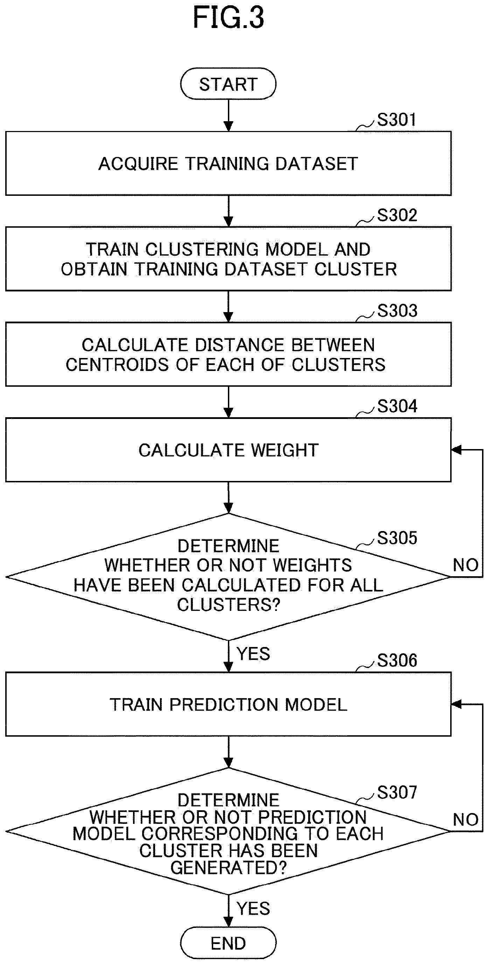

Next, a flow of a training process will be described. FIG. 3 is a flowchart illustrating the flow of the training process.

In Step S301, the prediction model generating device 120 acquires the training dataset 111.

In Step S302, the prediction model generating device 120 trains the clustering model 121, using the acquired training dataset 111, to generate the trained clustering model 131, and obtains the centroid coordinates between the clusters and the training dataset clusters classified into N clusters.

In Step S303, the weight defining part 122 calculates the distance $\{l_{ij}\}_{1 \leq i \leq N, 1 \leq j \leq N}$ between the centroids of the clusters for the training dataset cluster i.

In Step S304, the weight defining part 122 calculates the weight $\{W_{ij}\}_{1 \leq i \leq N, 1 \leq j \leq N}$ to be used in the prediction model 123, using the distance between the clusters and the parameter representing the feature of the training dataset 111.

In Step S305, the prediction model generating device 120 determines whether or not the weight have been calculated for all clusters of the training dataset 111 classified into N clusters. In a case where it is determined in Step S305 that there is any cluster whose weight has not been calculated (NO in Step S305), the process returns to Step S304.

In a case where it is determined in Step S305 that there is no cluster whose weight has not been calculated (YES in Step S305), the process proceeds to Step S306.

In Step S306, the prediction model generating device 120 trains the prediction model 123, using a combination of the generated training dataset cluster and the corresponding weight, to generate the trained prediction model 132.

In Step S307, the prediction model generating device 120 determines whether or not training of the prediction model 123 has been performed for all clusters of the training dataset 111 classified into N clusters. In a case where it is determined that there is any cluster for which the trained prediction model 132 is not generated in Step S306 (NO in Step S307), the process returns to Step S306.

In a case where it is determined in Step S307 that there is no training dataset cluster for which the trained prediction model 132 has not been generated (YES in Step S007), the training process is terminated.

<Flow of Prediction Process>

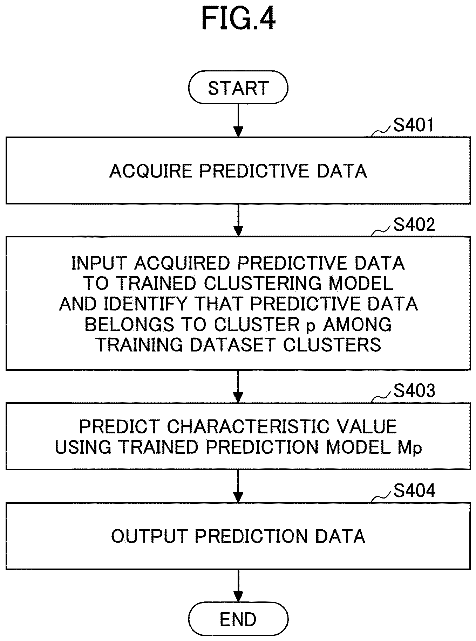

Next, a flow of a prediction process will be described. FIG. 4 is a flowchart illustrating the flow of the prediction process.

In Step S401, the prediction device 130 acquires predictive data (design condition x).

In Step S402, the prediction device 130 inputs the acquired predictive data to the trained clustering model 131, and identifies that the predictive data belongs to the cluster p among the training dataset clusters.

In Step S403, the prediction device 130 acquires the trained prediction model $132M_p$ corresponding to the cluster p, and predicts the characteristic value using trained prediction model with the acquired predictive data as the input.

In Step S404, the prediction device 130 outputs the predicted characteristic value, as prediction data for the input data (design condition x) of the prediction target.

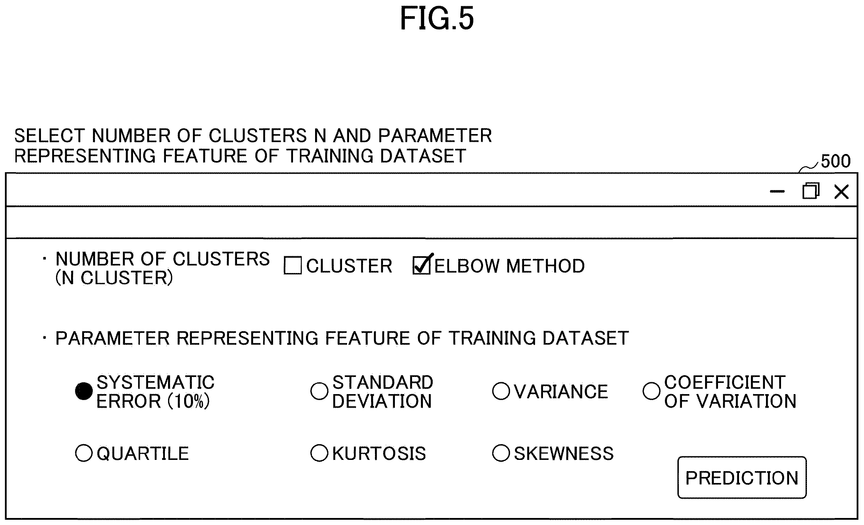

A screen 500 in FIG. 5 illustrates a graphical user interface (GUI) for setting the number of clusters N by selecting manual setting or elbow method automatic setting and for selecting a type of the parameter representing the feature of the training dataset 111, when generating the clustering model 121. The user sets the optimum number of clusters on the screen, and selects a parameter, such as systematic error, standard deviation, variance, coefficient of variation, quartile, kurtosis, or skewness. For example, FIG. 5 illustrates an example in which the elbow method automatic setting is selected as a method of setting the number of clusters, and the systematic error is selected as the parameter representing the feature of the training dataset 111. When the "prediction" button is pressed in the state of FIG. 5 described above, the clustering model 121, the weight defining part 122, and the prediction model 123 of the prediction model generating device 120 generate the trained clustering model 131 and the trained prediction model 132 according to the procedures of the flowchart of FIG. 3.

<Summary>

As is apparent from the above description, the prediction device 130 according to the embodiment

includes the trained clustering model 131 for clustering input data and the trained prediction model 132 corresponding to the cluster p, and

outputs, as prediction data, the characteristic value predicted by the trained prediction model 132, using the appropriate weight.

Thus, according to the prediction device 130 according to the embodiment, the prediction accuracy can be improved in the prediction device 130 using the trained prediction model 132.

[Example]

A specific example of the prediction method of the present disclosure will be described using a known dataset. Note that the characteristic prediction according to the present disclosure can be applied not only to the field of material but also to other fields.

In the description of the example, it is assumed that the material data storage part 110 stores, for example, 506 data of the Boston housing prices dataset disclosed in Toy datasets in the scikit-learn (https://scikit-learn.org/stable/datasets/toy_dataset.html).

In a case where the prediction model generating process and the prediction process are performed with the Boston housing prices dataset, the processes are performed by the following procedures.

[Training Procedures]

(1) Procedure 1

The training procedures will be described below.

The Boston house prices dataset was randomly divided into a training dataset/a predictive dataset in a ratio of 75%/25%. Among the Boston house prices dataset, explanatory variables include CRIM ("per capita crime rate" per town), ZN ("proportion of residential land zoned for big homes"), INDUS ("proportion of non-retail business acres" per town), CHAS ("by the river or not"), NOX ("NOx concentration (in 0.1 ppm)"), RM ("average number of rooms" per home), AGE ("proportion of old dwellings"), DIS ("distances to the major buildings"), RAD ("accessibility to highways"), TAX ("property-tax rate"), PTRATIO ("pupil-teacher ratio" per town), B ("population of blacks" per town), and LSTAT ("lower status of the population"), and a target variable includes MEDV (median value of "owner-occupied homes" (in $1000s)).

(2) Procedure 2

Using the training dataset acquired in Procedure 1, training was performed using the K-Means method which is a clustering algorithm stored in the scikit-learn to obtain a trained clustering model.

(3) Procedure 3

Training dataset clusters classified into N clusters were obtained using the trained clustering model trained in Procedure 2, when the training dataset is input. Here, two clusters were obtained by the elbow method.

(4) Procedure 4

The distance $\{l_{ij}\}_{1 \leq i \leq N, 1 \leq j \leq N}$ between the centroids of the clusters was calculated in $N(N-1)/2$ ways for the training dataset clusters classified in Procedure 3. Here, an Euclidean distance is applied as the distance between centroids of the clusters.

(5) Procedure 5

Using the distance $\{l_{ij}\}_{1 \leq i \leq N, 1 \leq j \leq N}$ calculated in Procedure 4 and the parameter representing the feature of the training dataset, a weight between the clusters $\{W_{ij}\}_{1 \leq i \leq N, 1 \leq j \leq N}$ were calculated. Here, the standard deviation of the MEDV of the training dataset was used as the parameter representing the feature of the training dataset. Furthermore, a weighting function represented by Equation (1) below was used as the weight between the clusters. Note that $\alpha=1.0$ was used as a predetermined constant.

[Math. 2]

$$ w_{ij} = \exp\left(\frac{-l_{ij}}{\tau^{\alpha}}\right) \quad \ldots (1) $$

where

$l_{ij}$ denotes a distance between the clusters calculated in Procedure 4,
$\tau$ denotes a parameter representing the feature of the dataset, and

$\alpha$ denotes a predetermined constant.

(6) Procedure 6

Using the random forest regression algorithm stored in the scikit-learn as a prediction model, for each cluster, a prediction model $M_i$ was trained with the training dataset cluster generated in Procedure 2 and the corresponding weight for the cluster generated in Procedure 5. Thus, two trained prediction models were obtained. In such a case, as a training method to which a weight is applied, a weight was input to a parameter of the fit function of the random forest regression algorithm stored in the scikit-learn.

[Prediction Procedures]

(7) Procedure 7

The prediction procedures will be described below.
Predictive data was acquired from the predictive dataset acquired in Procedure 1. Using the trained clustering model trained in Procedure 2, it was identified whether the predictive data belongs to a cluster p among the clusters described in Procedure 3.

(8) Procedure 8

Using the predictive data as an input, a characteristic value was predicted using a trained prediction model $M_p$ corresponding to the cluster p to which the predictive data belongs, the trained prediction model $M_p$ having been generated in Procedure 6. The predicted characteristic value was then output as prediction data.
Each of the remaining predictive data of the predictive dataset was processed in the same manner, and the respective pieces of prediction data were output.

(9) Procedure 9

The prediction accuracy of the prediction method of the present disclosure was determined. A $R^2$ value defined by Equation (2) below was used as an evaluation indicator for the prediction accuracy. The closer the $R^2$ value is to 1, the higher the prediction accuracy is.
[Math. 3]

$$R^2(y, \hat{y}) = 1 - \frac{\sum_{i=1}^{n}(y_i - \hat{y}_i)^2}{\sum_{i=1}^{n}(y_i - \bar{y})^2} \qquad \dots (2)$$

where

$\hat{y}_i$     denotes a prediction value of ith predictive data,
$y_i$     denotes an actual value of ith predictive data,
$\bar{y}$     denotes an average value of the predictive data, and
n     denotes a number of pieces of data of the predictive data.

[Comparative Example]

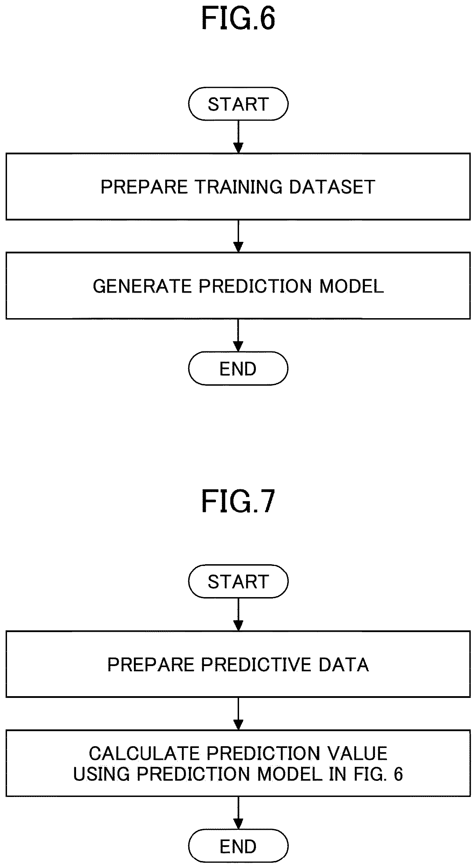

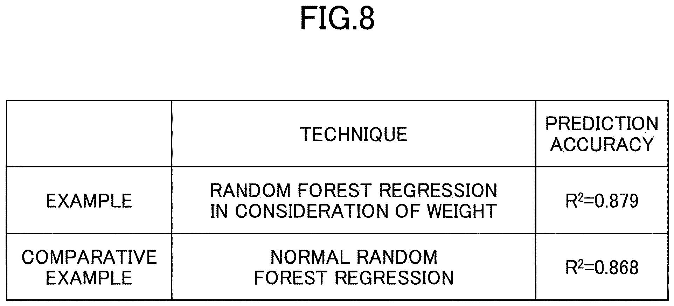

In a comparative example, a characteristic value was predicted using the prediction model in the same manner as in the example, and the $R^2$ value was calculated, as illustrated in the flowcharts in FIG. 6 and FIG. 7 except that the clustering in Procedure 2 was not performed and the weight in Procedure 5 was not used.
As the prediction accuracy of the example, $R^2 = 0.879$ was obtained. On the other hand, $R^2 = 0.868$ was obtained as the prediction accuracy of the comparative example.
As illustrated in FIG. 8, it can be seen that the prediction accuracy of the random forest regression model in which, as in the present embodiment, cluster classification is performed and an appropriate weight is taken into consideration is higher than the prediction accuracy of the normal random forest regression model in the comparative example.
As described above, by classifying the predictive data into appropriate clusters and constructing a model in which an appropriate weight is considered for each cluster, prediction can be performed with higher accuracy than that in the comparative example.

[Other Embodiment]

In the embodiment described above, the prediction model generating device and the prediction device have been described as separate devices. However, the prediction model generating device and the prediction device may be configured as an integrated device.

Note that, in the above-described embodiment, the distance between the centroids is calculated using the Euclidean distance, and other specific examples for calculating the distance are not described. The method of calculating the distance between the centroids may be, for example, the Manhattan distance method, Mahalanobis distance method, Minkowski distance method, cosine distance method, shortest distance method, longest distance method, centroid method, group average method, Ward's method, Kullback-Leibler divergence, Jensen-Shannon divergence, Dynamic time warping, Earth mover's distance, or the like.

In the above-described embodiment, the training is performed using the K-Means method and the random forest regression algorithm, and specific examples of other training techniques are not mentioned. The training technique used when training the clustering model may be, for example, the Nearest Neighbor method, hierarchical clustering method, Gaussian mixture method, DBSCAN method, t-SNE method, self-organizing map method, or the like.

The training technique used when training the prediction model may be, for example, the decision tree, gradient boosting, AdaBoost, bagging, linear, partial least squares, Lasso, linear ridge, elastic net, or the like.

In one embodiment of the present disclosure, design conditions of a material whose characteristics have been predicted by the prediction method of the present disclosure can also be used for manufacturing. For example, the device for manufacturing the material can acquire, from the prediction device 130, the information on the design conditions of the material whose characteristics are predicted by the prediction device 130, and manufacture the material using the acquired information on the design conditions.

The present invention is not limited to the configurations of the embodiments described above, and the configurations may be combined with other elements. In this respect, variations may be made without departing from the scope of the present disclosure, and the variations may be determined appropriately according to the applications.

DESCRIPTION OF THE REFERENCE NUMERALS

111    training dataset
120    prediction model generating device
121    clustering model
122    weight defining part
123    prediction model
130    prediction device
131    trained clustering model
132    trained prediction model
133    output part

**Claims**

1. A method of generating a model for predicting a material characteristic, the method comprising steps of:

    acquiring a training dataset;
    generating a trained clustering model, using the training dataset and a clustering model, and classifying the training dataset into N clusters;
    calculating a distance between centroids of the clusters;
    calculating a weight between the clusters, using both the distance between the centroids of the clusters and a parameter representing a feature of the training dataset; and
    generating, for the N clusters, respective trained prediction models $\{M_i\}_{1 \leq i \leq N}$, using the clusters and the weight.

2. A prediction method of a material characteristic, the prediction method being performed subsequent to the method of generating a model for predicting a material characteristic as claimed in claim 1 and comprising steps of:

acquiring data used for the predicting;

identifying, using the trained clustering model, that the data used for the predicting belongs to a cluster p among training dataset clusters classified into N pieces; and

determining a prediction value, using a trained prediction model $M_p$ corresponding to the cluster p with the data used for the predicting as an input.

3. The method of generating a model for predicting a material characteristic as claimed in claim 1, wherein, in the step of generating a clustering model of claim 1, at least one or a plurality of clustering techniques among a K-means method, a Nearest Neighbor method, a hierarchical clustering method, a Gaussian mixture method, a DBSCAN method, a t-SNE method, and a self-organizing map method is used.

4. The method of generating a model for predicting a material characteristic as claimed in claim 1, wherein, in the step of calculating a distance between centroids of the clusters of claim 1, the distance is calculated using at least one or a combination of a plurality of methods among an euclidean distance method, a Manhattan distance method, a Mahalanobis distance method, a Minkowski distance method, a cosine distance method, a shortest distance method, a longest distance method, a centroid method, a group average method, a Ward's method, a Kullback-Leibler divergence, a Jensen-Shannon divergence, a Dynamic time warping, and an Earth mover's distance.

5. The method of generating a model for predicting a material characteristic as claimed in claim 1, wherein, as the parameter representing the feature of the training dataset, at least one or a plurality of parameters among a systematic error, a standard deviation, a variance, a coefficient of variation, a quantile, kurtosis, and a skewness related to a characteristic value of the training dataset is used.

6. The method of generating a model for predicting a material characteristic as claimed in claim 1, wherein, in the step of calculating a weight, at least one or a plurality of weighting functions among an exponential function type, a reciprocal function type, and a reciprocal power type is used.

7. A device of generating a model for predicting a material characteristic, the device comprising:

a clustering model configured to generate a trained clustering model when a training dataset is input, and classify the training dataset into N clusters; and

a weight defining part configured to calculate a distance between centroids of the clusters of the classified clusters, and a weight between the clusters, using both the calculated distance between the centroids of the clusters and a parameter representing a feature of the training dataset; and

a prediction model $\{M_i\}_{1 \le i \le N}$ configured to generate, for the clusters, respective trained prediction models, using the clusters and the weight.

8. A device of predicting a material characteristic, the device comprising:

the trained clustering model generated by the device of generating a model for predicting a material characteristic as claimed in claim 7, the trained clustering model being configured to identify, when data used for the predicting is input, that the data used for the predicting belongs to a cluster p among clusters classified into N pieces;

a trained prediction model $M_p$ corresponding to the identified cluster p and generated by the device of generating a model for predicting a material characteristic as claimed in claim 7, the trained prediction model $M_p$ being configured to determine a prediction value with data used for the predicting as an input; and

an output part configured to output the determined prediction value.

9. A program of generating a model for predicting a material characteristic, the program causing a computer to execute steps of:

acquiring a training dataset;

generating a trained clustering model, using the training dataset and a clustering model, and classifying the training dataset into N clusters;

calculating a distance between centroids of the clusters;

calculating a weight between the clusters, using both the distance between the centroids of the clusters and a parameter representing a feature of the training dataset; and

generating, for the clusters, respective trained prediction models $\{M_i\}_{1 \le i \le N}$, using the clusters and the weight.

10. A program of predicting a material characteristic, the program causing a computer to execute steps of:

acquiring data used for the predicting;
identifying, using the trained clustering model generated by the program of generating a model for predicting as claimed in claim 9, that the data used for the predicting belongs to a cluster p among training dataset clusters classified into N pieces; and
determining, when the data used for the predicting is input, a prediction value, using a prediction model $M_p$ as claimed in claim 9, the prediction model $M_p$ corresponding to the identified cluster p.

# FIG.1

(a)

MATERIAL DATA STORAGE PART ~110

TRAINING DATASET ~111

| INPUT DATA | CORRECT ANSWER DATA |
|---|---|
| DESIGN CONDITION 1 | CHARACTERISTIC VALUE 1 |
| DESIGN CONDITION 2 | CHARACTERISTIC VALUE 2 |
| ... | ... |
| DESIGN CONDITION n | CHARACTERISTIC VALUE n |

PREDICTION MODEL GENERATING DEVICE ~120

| CLUSTERING MODEL ~121 | → | WEIGHT DEFINING PART ~122 | → | PREDICTION MODEL ~123 |
|---|---|---|---|---|

(b)

PREDICTION DEVICE ~130

DESIGN CONDITION x →

| TRAINED CLUSTERING MODEL ~131 | → | TRAINED PREDICTION MODEL ~132 | → | OUTPUT PART ~133 |
|---|---|---|---|---|

→ CHARACTERISTIC VALUE y

EP 4 411 602 A1

# FIG.2

~120, 130

PREDICTION MODEL GENERATING
DEVICE AND PREDICTION DEVICE

~201

PROCESSOR

~202

MEMORY

~203

AUXILIARY
STORAGE
DEVICE

~207

~204

I/F DEVICE

~205

COMMUNICATION
DEVICE

~206

DRIVE DEVICE

~110

MATERIAL DATA
STORAGE PART

~210

RECORDING
MEDIUM

# FIG.3

```
                    ( START )
                         │
                         ▼              S301
    ┌────────────────────────────────────────────┐
    │         ACQUIRE TRAINING DATASET            │
    └────────────────────────────────────────────┘
                         │
                         ▼              S302
    ┌────────────────────────────────────────────┐
    │        TRAIN CLUSTERING MODEL AND           │
    │      OBTAIN TRAINING DATASET CLUSTER        │
    └────────────────────────────────────────────┘
                         │
                         ▼              S303
    ┌────────────────────────────────────────────┐
    │        CALCULATE DISTANCE BETWEEN           │
    │      CENTROIDS OF EACH OF CLUSTERS          │
    └────────────────────────────────────────────┘
                         │
                         ▼              S304
    ┌────────────────────────────────────────────┐
    │             CALCULATE WEIGHT                │◄──┐
    └────────────────────────────────────────────┘   │
                         │                            │
                         ▼              S305          │
                  ╱DETERMINE╲                         │
              ╱ WHETHER OR NOT WEIGHTS ╲      NO       │
             ╱  HAVE BEEN CALCULATED FOR ALL ╲─────────┘
              ╲        CLUSTERS?      ╱
                  ╲             ╱
                         │ YES
                         ▼              S306
    ┌────────────────────────────────────────────┐
    │           TRAIN PREDICTION MODEL            │◄──┐
    └────────────────────────────────────────────┘   │
                         │                            │
                         ▼              S307          │
                  ╱DETERMINE╲                         │
              ╱WHETHER OR NOT PREDICTION╲     NO       │
             ╱MODEL CORRESPONDING TO EACH╲────────────┘
              ╲    CLUSTER HAS BEEN   ╱
                ╲   GENERATED?    ╱
                         │ YES
                         ▼
                    ( END )
```

# FIG.4

START

↓ S401

ACQUIRE PREDICTIVE DATA

↓ S402

INPUT ACQUIRED PREDICTIVE DATA
TO TRAINED CLUSTERING MODEL
AND IDENTIFY THAT PREDICTIVE DATA
BELONGS TO CLUSTER p AMONG
TRAINING DATASET CLUSTERS

↓ S403

PREDICT CHARACTERISTIC VALUE
USING TRAINED PREDICTION MODEL Mp

↓ S404

OUTPUT PREDICTION DATA

↓

END

# FIG.5

SELECT NUMBER OF CLUSTERS N AND PARAMETER
REPRESENTING FEATURE OF TRAINING DATASET

~500

— ◰ ✕

· NUMBER OF CLUSTERS ☐CLUSTER ☑ELBOW METHOD
(N CLUSTER)

· PARAMETER REPRESENTING FEATURE OF TRAINING DATASET

⬤ SYSTEMATIC        ○ STANDARD        ○ VARIANCE        ○ COEFFICIENT
  ERROR (10%)         DEVIATION                             OF VARIATION

○ QUARTILE          ○ KURTOSIS       ○ SKEWNESS

PREDICTION

EP 4 411 602 A1

# FIG.6

```
START
  │
  ▼
PREPARE TRAINING DATASET
  │
  ▼
GENERATE PREDICTION MODEL
  │
  ▼
END
```

# FIG.7

```
START
  │
  ▼
PREPARE PREDICTIVE DATA
  │
  ▼
CALCULATE PREDICTION VALUE
USING PREDICTION MODEL IN FIG. 6
  │
  ▼
END
```

# FIG.8

| | TECHNIQUE | PREDICTION ACCURACY |
|---|---|---|
| EXAMPLE | RANDOM FOREST REGRESSION IN CONSIDERATION OF WEIGHT | $R^2=0.879$ |
| COMPARATIVE EXAMPLE | NORMAL RANDOM FOREST REGRESSION | $R^2=0.868$ |

## INTERNATIONAL SEARCH REPORT

| | International application No. |
|---|---|
| | **PCT/JP2022/034047** |

**A. CLASSIFICATION OF SUBJECT MATTER**

*G06N 20/00*(2019.01)i; *G16C 20/70*(2019.01)i; *G16C 60/00*(2019.01)i
FI: G06N20/00; G16C20/70; G16C60/00

According to International Patent Classification (IPC) or to both national classification and IPC

**B. FIELDS SEARCHED**

Minimum documentation searched (classification system followed by classification symbols)

G06N20/00; G16C20/70; G16C60/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2020/261449 A1 (NIPPON TELEGRAPH AND TELEPHONE CORPORATION) 30 December 2020 (2020-12-30)<br>entire text, all drawings | 1-10 |
| A | JP 2004-086896 A (FUJI ELECTRIC HOLDINGS CO., LTD.) 18 March 2004 (2004-03-18)<br>entire text, all drawings | 1-10 |
| A | JP 2020-533700 A (NEC CORPORATION) 19 November 2020 (2020-11-19)<br>entire text, all drawings | 1-10 |

☐ Further documents are listed in the continuation of Box C.    ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/034047**

| Patent document cited in search report | | | Publication date (day/month/year) | Patent family member(s) | | | Publication date (day/month/year) |
|---|---|---|---|---|---|---|---|
| WO | 2020/261449 | A1 | 30 December 2020 | (Family: none) | | | |
| JP | 2004-086896 | A | 18 March 2004 | (Family: none) | | | |
| JP | 2020-533700 | A | 19 November 2020 | US<br>entire text, all drawings<br>WO | 2020/0311574<br><br>2019/064598 | A1<br><br>A1 | |

Form PCT/ISA/210 (patent family annex) (January 2015)

**EP 4 411 602 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021159474 A
- JP 2020187417 A